# EUROPEAN PATENT SPECIFICATION

(11) **EP 0 512 656 B1**
(45) Date of publication and mention of the grant of the patent: **10.04.1996**
(21) Application number: 92201337.0
(22) Date of filing: 08.05.1992
(51) Int. Cl.: C07C 69/01, C07C 67/04, C07D 207/267

(54) **Process for the preparation of vinyl derivatives**
Verfahren zur Herstellung von Vinylderivaten
Procédé de préparation de dérivés vinyliques

(30) Priority: 10.05.1991 GB 9110110
(43) Date of publication of application: 11.11.1992
(73) Proprietor: SHELL INTERNATIONALE RESEARCH MAATSCHAPPIJ B.V., NL-2596 HR Den Haag (NL)
(72) Inventor: Drent, Eit, NL-1031 CM Amsterdam (NL)

(56) References cited:
- JOURNAL OF ORGANIC CHEMISTRY. vol. 52, 1987, EASTON US pages 2230 - 2239 TAKE-AKI MUTSIDO ET AL. 'RUTHENIUM-CATALIZED SELECTIVE ADDITION OF CARBOXYLIC ACIDS TO ALKYNES.A NOVEL SYNTHESIS OF ENOL ESTERS'

## Description

This invention relates to a process for the preparation of a vinyl derivative of a Bronsted acid. "Vinyl" is defined here as an ethenyl radical optionally substituted at the 1- and/or 2-positions, and the "Bronsted acid" is a compound which can release protons. Typically, vinyl derivatives of Bronsted acids include ethenyl esters of carboxylic acids and ethenyl ethers of hydroxy compounds, which find extensive commercial use, e.g., as monomers in free radical initiated addition polymerizations.

Processes for the preparation of such vinyl derivatives by addition of a Bronsted acid to an alkyne are known for a long time, and are typically catalyzed by a Group 2b metal compound, for example a carboxylate of zinc, cadmium or mercury. These known processes generally suffer from the disadvantage of loss of catalytically active metal in the course of time. Whether using the Group 2b metal catalyst in homogeneous or heterogeneous form, adequate measures are to be taken to prevent a quick loss of catalyst activity, as shown, for example, by GB-A-1125055, DE-A-1233387 and US 3652659. Nevertheless, it has appeared to be difficult to effect high confinement or recovery of the metallic catalyst component in practice. Moreover, the catalysis by Group 2b metal compounds requires a relatively high load of catalytically active metal, whilst for attractive reaction rates quite high reaction temperatures are required.

The present invention aims at providing an improved catalyst for the above reaction avoiding or at least mitigating the described problems. More particularly, the invention aims at providing an improved supported catalyst allowing the reaction to be conducted in the gaseous phase.

It should be remarked, that a process for the preparation of a vinyl derivative of a Bronsted acid is known from EP-A-351603, according to which a vinyl derivative of a Bronsted acid is transvinylated with a different Bronsted acid in the presence of a ruthenium compound. This process starts from different precursors, and the separation of the several Bronsted acids and their derivatives may be cumbersome. M. Rotem et al., Organometallics, 2, pp. 1689-91 (1983) disclose the addition of carboxylic acids to substituted ethynes using unsupported ruthenium compound catalysts. Despite the higher reactivity of substituted ethynes as compared with unsubstituted ethyne, they observed acceptable conversions at reaction times as high as 17 to 22 hours. Besides, the ruthenium complexes used are not suitable for a continuous gas phase process over a contained catalyst bed.

The use of ruthenium(II)-containing catalysts, viz. bis(η⁵-cyclooctadienyl)ruthenium(II)(trialkylphosphine)(maleic anhydride), is disclosed by T. Mitsudo et al. in J. Org. Chem. (1987), 52, pp. 2230-2239. For the addition of unsaturated carboxylic acids, the presence of maleic acid in the catalyst system is not essential, but in the absence of maleic acid no reaction was observed with saturated carboxylic acids. Whereas the recorded reaction times are shorter (4 hours), the yields obtained show, that the reaction proceeds at relatively low catalyst turnover rate.

An advantageous process for the preparation of vinyl derivatives of Bronsted acids has now been found by the inventor, which process involves contacting an acetylenically unsaturated compound and a Bronsted acid at elevated temperatures in the presence of a heterogeneous catalyst comprising elemental ruthenium supported on an inert porous support.

It has been found that using the supported ruthenium catalyst high conversion rates are achieved in the addition reaction of a Bronsted acid to an acetylenically unsaturated compound, in particular to ethyne. Moreover, the catalysts to be used according to the invention show no tendency of sublimation with consequent loss of catalytic activity and problematic need of recovery of the lost metal. The supported ruthenium catalysts are advantageously suitable for conducting the present process in the gaseous phase.

Bronsted acids typically used in the process according to the invention include carboxylic acids such as acetic acid, propionic acid, butyric acid, pivalic acid and other trialkylacetic acids, stearic acid, benzoic acid, the phthalic acids, adipic acid, succinic acid, maleic acid, acrylic acid and methacrylic acid; alcohols such as methanol, ethanol, isopropanol, phenol, hydroquinone and resorcinol; amino compounds of sufficient acidity, such as secondary aromatic amines and primary aliphatic amines; hydroxy esters such as hydroxy alkyl acrylates and hydroxy alkyl alkanoates; and amides or imides such as 2-pyrrolidone, ε-caprolactam, succinimide and toluenesulphonamide. Preferred Bronsted acids are selected from the group of carboxylic acids, cyclic amides and aromatic hydroxy compounds, more preferably from the group of aliphatic carboxylic acids having 1-30 carbon atoms. A most preferred group of aliphatic carboxylic acids is constituted by the trialkylacetic acids.

Representative addition product of the present process include vinyl acetate, vinyl propionate, vinyl butyrate, vinyl pivalate, vinyl neoheptanoate, vinyl neodecanoate, vinyl benzoate, vinyl acrylate, vinyl methacrylate, divinyl terephthalate, divinyl adipate, N-vinyl succinimide, N-vinyl 2-pyrrolidone, vinyl phenyl ether, vinyl methyl ether, vinyl butyl amine, 2-vinyloxyethyl acetate and N-vinyl toluenesulphonamide.

The catalyst utilised in accordance with the present invention comprises elemental ruthenium supported on an inert porous support. The supported ruthenium catalyst is particularly advantageous in the process of the instant invention as it is readily separated from the feed and product. Suitable inert porous supports include refractory oxides and carbon. Examples of suitable refractory oxides include alumina, silica, silica-alumina, titania, zirconia, magnesia and the like. Preferred inert porous supports for use in the instant catalysts are alumina and carbon.

The catalysts which are utilised in the present invention can be suitably prepared using any conventional technique such as, for example, impregnation, coprecipitation, comilling, spray drying and the like; or any combination of these conventional techniques.

The present reaction may be carried out batch-wise or continuously. For example it may be carried out in a fixed bed reactor, the bed comprising the catalyst, wherein the feed of acetylenically unsaturated compound and Bronsted acid is passed over the bed. The feed may be passed in liquid or gaseous form over the catalyst bed. Other continuous reactor configurations will be readily apparent to one skilled in the art. In a batch mode, a gaseous feed of the acetylenically unsaturated compound may be introduced into an agitated mixture of the Bronsted acid and the supported catalyst. Any of the gaseous or liquid feeds or reaction mixtures may be diluted with inert gases or solvents. Further batch reactor configurations will be apparent to the skilled artisan.

The present reaction proceeds at a molar ratio between acetylenically unsaturated compound and Bronsted acid of 1:1. However, the feed may comprise either of the reactants in excess. For example, the excess of one of the reactants may function as a carrier for the reaction product for ease of separation of the latter. An excess of acetylenically unsaturated compound may be beneficial in suppressing any undesired occurrence of a repeated addition of the Bronsted acid to the vinyl derivative reaction product. The molar ratio between the acetylenically unsaturated compound and the Bronsted acid may range from 100:1 to 1:100, more typically from 10:1 to 1:10.

The process of the invention is carried out at elevated temperatures. The reaction temperature is typically in the range of 40-250 °C, preferably in the range of 80-175 °C. Reaction pressures for the present process are not critical and will typically be atmospheric. However, the reaction pressure may also be subatmospheric, for example for volatilising high boiling reactants, or superatmospheric, in so far as compatible with the safety requirements regarding acetylenically unsaturated compounds.

The invention will be further illustrated by the following examples.

### Example 1

After being flushed, a 250 ml autoclave was charged with 10 ml of acetic acid, 40 ml of diglyme (2,5,8-trioxanonane), 1.4 bar of ethyne and 1 g of a ruthenium (5 %wt) on carbon catalyst. For safety considerations, 20 bar of nitrogen gas was added. Upon sealing, the agitated contents of the autoclave were heated to 140 °C for 1.5 hour. Thereupon the autoclave was cooled and its content analysed by means of standards gas liquid chromatography (GLC). It was found that 80 % of the ethyne was converted. The selectivity into vinyl acetate was found to be 82%.

### Example 2

Example 1 was repeated using 10 ml of pivalic acid instead of 10 ml of acetic acid. Upon a reaction time of 2 hours, GLC analysis showed an ethyne conversion of 80% with a selectivity to vinyl pivalate of 71%.

### Comparative Experiments

Example 1 was repeated using 1 g of rhodium (5 %wt) on carbon, platinum (5 %wt) on carbon, and palladium (10 %wt) on carbon, respectively instead of the ruthenium catalyst. Upon a reaction time of 8 hours, none of the experiments showed a conversion of acetylene higher than 10%. These comparative experiments demonstrate the unique suitability of supported ruthenium catalysts amongst the Group VIII metals.

### Example 3

After being flushed, a 250 ml autoclave was charged with 10 ml of 2-pyrrolidone, 50 ml of diglyme (2,5,8-trioxanonane), 1.5 bar of ethyne and 1 g of a ruthenium (5 %wt) on carbon catalyst. For safety considerations, 20 bar of nitrogen gas was added. Upon sealing, the agitated contents of the autoclave were heated to 170 °C for 7 hours. Thereupon the autoclave was cooled and its content analysed by means of standards gas liquid chromatography (GLC). It was found that 90% of the ethyne was converted. The selectivity into N-vinyl 2-pyrrolidone was found to be above 95%.

## Claims

1. A process for the preparation of a vinyl derivative of a Bronsted acid by contacting an acetylenically unsaturated compound and a Bronsted acid at elevated temperatures in the presence of a heterogeneous catalyst comprising elemental ruthenium supported on an inert porous support.

2. A process as claimed in claim 1, wherein the acetylenically unsaturated compound is ethyne.

3. A process as claimed in any one or more of claims 1-2, wherein the Bronsted acid is selected from the group of carboxylic acids, cyclic amides and aromatic hydroxy compounds.

4. A process as claimed in claim 3, wherein the carboxylic acid is an aliphatic carboxylic acid having 1 -30 carbon atoms.

5. A process as claimed in claim 4, wherein the aliphatic carboxylic acid is a trialkylacetic acid.

6. A process as claimed in any one or more of claims 1-5, wherein the temperature is in the range of 40-250 °C, preferably in the range of 80-175 °C.

7. A process as claimed in any one or more of claims 1-6, wherein the inert porous support is selected from the group of refractory oxides and carbon.

8. A process as claimed in claim 7, wherein the refractory oxide is selected from the group of alumina, silica, silica-alumina, titania, zirconia, magnesia and mixtures thereof.

9. A process as claimed in claim 7, wherein the inert porous support is carbon.

## Patentansprüche

1. Verfahren zur Herstellung eines Vinylderivats einer Brönstedsäure durch Inberührungbringen einer acetylenisch ungesättigten Verbindung mit einer Brönstedsäure bei erhöhten Temperaturen in Gegenwart eines heterogenen Katalysators, der auf einem inerten, porösen Träger fixiertes elementares Ruthenium enthält.

2. Verfahren nach Anspruch 1, bei dem man als acetylenisch ungesättigte Verbindung Ethin einsetzt.

3. Verfahren nach einem der Ansprüche 1-2, bei dem man die Brönstedsäure aus der Gruppe der Carbonsäuren, cyclischen Amide und aromatischen Hydroxyverbindungen auswählt.

4. Verfahren nach Anspruch 3, bei dem man als Carbonsäure eine aliphatische Carbonsäure mit 1-30 Kohlenstoffatomen einsetzt.

5. Verfahren nach Anspruch 4, bei dem man als aliphatische Carbonsäure eine Trialkylessigsäure einsetzt.

6. Verfahren nach einem der Ansprüche 1-5, bei dem man eine Temperatur im Bereich von 40-250°C, vorzugsweise im Bereich von 80-175°C, einhält.

7. Verfahren nach einem der Ansprüche 1-6, bei dem man den inerten, porösen Träger aus der Gruppe der feuerfesten Oxide und Kohlenstoff auswählt.

8. Verfahren nach Anspruch 7, bei dem man das feuerfeste Oxid aus der Gruppe Aluminiumoxid, Siliziumdioxid, Siliziumdioxid-Aluminiumoxid, Titandioxid, Zirconiumdioxid, Magnesiumoxid und deren Gemischen auswählt.

9. Verfahren nach Anspruch 7, bei dem man als inerten, porösen Träger Kohlenstoff einsetzt.

## Revendications

1. Procédé de préparation d'un dérivé vinylique d'un acide de Bronsted par la mise en contact d'un composé à insaturation acétylénique et d'un acide de Bronsted à des températures élevées et en présence d'un catalyseur hétérogène constitué de ruthénium élémentaire porté par un support poreux inerte.

2. Procédé suivant la revendication 1, caractérisé en ce que le composé à insaturation acétylénique est l'éthyne.

3. Procédé suivant la revendication 1 ou 2, caractérisé en ce que l'on choisit l'acide de Bronsted dans le groupe formé par les acides carboxyliques, les amides cycliques et les composés aromatiques hydroxylés.

4. Procédé suivant la revendication 3, caractérisé en ce que l'acide carboxylique est un acide carboxylique aliphatique comportant de 1 à 30 atomes de carbone.

5. Procédé suivant la revendication 4, caractérisé en ce que l'acide carboxylique aliphatique est un acide trialkylacétique.

6. Procédé suivant l'une quelconque des revendications 1 à 5, caractérisé en ce que la température varie de 40 à 250°C, de préférence de 80 à 175°C.

7. Procédé suivant l'une quelconque des revendications 1 à 6, caractérisé en ce que l'on choisit le support poreux inerte dans le groupe formé par les oxydes réfractaires et le carbone.

8. Procédé suivant la revendication 7, caractérisé en ce que l'on choisit l'oxyde réfractaire dans le groupe formé par l'alumine, la silice, la silice-alumine, l'oxyde de titane, l'oxyde de zirconium, l'oxyde de magnésium et leurs mélanges.

9. Procédé suivant la revendication 7, caractérisé en ce que le support poreux inerte est le carbone.
